# EUROPEAN PATENT APPLICATION

(11) **EP 3 991 691 A1**
(43) Date of publication of application: **04.05.2022**
(21) Application number: 20832351.9
(22) Date of filing: 05.06.2020
(51) Int. Cl.: A61D 13/00

(54) **BIO-INFORMATION MEASUREMENT GARMENT FOR ANIMAL, AND BIO-INFORMATION MEASUREMENT METHOD**

(30) Priority: 27.06.2019 JP 2019119468
(71) Applicant: TOYOBO CO., LTD., Osaka-shi Osaka 530-8230 (JP)
(72) Inventor: KOMATSU, Yoko, Otsu-shi, Shiga 520-0292 (JP); OMOTE, Yuichiro, Osaka-shi, Osaka 530-8230 (JP)
(74) Representative: Müller-Boré & Partner Patentanwälte PartG mbB
(86) International application number: PCT/JP2020/022370
(87) International publication number: WO 2020/261943

(57) **Abstract**

An object of the present invention is to provide a garment for measuring biological information for animals, in which the electrode portion is less likely to be displaced from the body even upon a movement of an animal and the biological information can be measured accurately, and to provide a method for measuring biological information.

A garment for measuring biological information comprising a front body and a back body having a large tensile elongation EMT. Since the garment for measuring biological information has a band around a torso, it is possible to prevent the electrodes from being displaced from the body due to a movement. Further, since the connection portion between the front body and the back body of the garment for measuring biological information is made of a woven or knitted fabric that expands and contracts in the direction from the head to the tail, the electrodes are less likely to be displaced from the body even upon a great movement. Therefore, the garment for measuring biological information of the present invention can accurately measure the biological information of a moving animal.

## Description

### TECHNICAL FIELD

The present invention relates to: a garment for measuring biological information for animals, in which an electrode section is less likely to be displaced from the body of an animal dressed in the garment even upon a vehement and great movement of the animal so that biological information can be measured with high accuracy; and a biological information measurement method.

### BACKGROUND ART

In recent years, the number of owners who place importance on the health of their pets has increased, and the need for easily acquiring biological information of their pets has increased. When a sensor (electrode) for monitoring biological information is closely attached to the body of an animal covered with hair, such as dogs, cats, rabbits, goats, miniature pigs, or horses, the owner needs to split the hair as much as possible or to trim the hair. Patent Document 1 discloses an invention of a jacket which can satisfactorily perform measurement on an electrocardiogram for a long time without giving a feeling of discomfort to dogs. Specifically, in the jacket, an electrode is attached to a detection site using an adhesive pad, and further, the electrode is appropriately pressed by externally applying a force using a fastening tool, in order to eliminate a need to trim an area where a sensor (electrode) for monitoring biological information is to be attached, or in order that it is only necessary to trim the minimum area approved by an owner or the like even if trimming is required.

Patent Document 2 discloses an invention of a wearable instrument that can measure biological information for a long time by pressing an electrode against the axilla of an animal. In the biological information measurement instrument, the length of a pull-up band shaped like a strap can be adjusted so that a measurement section is in close contact with the axilla of the animal. When the instrument is put on a dog, the measurement section is likely to be easily in close contact with the body, because the density of hair is usually low on the axilla. However, in both inventions, the electrode is placed in the lower part of the chest, such as the axilla, so that, when the dog is lying on its belly, the electrode or device is sandwiched between the dog and the floor and is strongly pressed against the body. This arises the problems that the electrode is likely to be displaced, the dog may express a feeling of discomfort, and the skin in the area where the electrode and the device are in strong contact may be damaged.

### RELATED ART DOCUMENTS

### PATENT DOCUMENTS

Patent Document 1: JP-A-2006-141467
Patent Document 2: WO2017/026416

### DISCLOSURE OF THE INVENTION

### PROBLEMS TO BE SOLVED BY THE INVENTION

The present invention has been made in view of the problems of prior arts described above. Specifically, an object of the present invention is to provide: a garment for measuring biological information for animals, in which a front body and a back body are easily elongated owing to high tensile elongations thereof to make it less likely for an electrode section to be displaced from the body even upon a great movement so that biological information can be measured with high accuracy; and a biological information measurement method.

### SOLUTIONS TO THE PROBLEMS

As a result of intensive studies, the present inventors have found that the above problems can be solved by the following means, and have reached the present invention. That is, the present invention includes the following configurations.
[1] A garment for measuring biological information for animals, the garment comprising
   a body contact electrode,
   a front body of the garment for measuring biological information, and
   a back body of the garment for measuring biological information, wherein
      the front body and/or the back body has a longitudinal-direction tensile elongation EMT of not lower than 5% and not higher than 110% and has a lateral-direction tensile elongation EMT of not lower than 10% and not higher than 130%.
[2] The garment for measuring biological information according to [1], wherein
   the front body and the back body of the garment for measuring biological information are connected to each other at a connection portion comprising a stretchable woven or knitted fabric.
[3] The garment for measuring biological information according to [2], wherein
   the woven or knitted fabric has an elongation percentage in a direction from a head portion to a tail portion, the elongation percentage being not lower than 100%.
[4] The garment for measuring biological information according to any one of [1] to [3], wherein
   the woven or knitted fabric is disposed at at least one portion of the garment for measuring biological information, the one portion being selected from among a shoulder portion, a shoulder joint portion, and an arm portion.
[5] The garment for measuring biological information according to any one of [1] to [4], wherein
   the body contact electrode is located on a side of the back body of the garment for measuring biological information, and the side is brought into contact with a body.
[6] The garment for measuring biological information according to any one of [1] to [5], the garment further comprising
   a band, wherein
   the band has a width of not smaller than 1 cm and not larger than 30 cm, and the band is attached in a direction around a torso of an animal.
[7] The garment for measuring biological information according to [6], wherein
   the band is disposed on an outer side of the garment at a location of the body contact electrode.
[8] The garment for measuring biological information according to any one of [1] to [7], wherein
   the body contact electrode is located at a thorax in the garment for measuring biological information.
[9] The garment for measuring biological information according to any one of [1] to [8], wherein
   biological information to be measured is an R-R interval which is an interval between an R wave and an R wave in an electrocardiogram.
[10] A biological information measurement method comprising dressing an animal in the garment for measuring biological information according to any one of [1] to [9], to measure biological information.
[11] The biological information measurement method according to [10], further comprising applying a conductive base to the body contact electrode.

### EFFECTS OF THE INVENTION

The present invention is used for measuring biological information by a wearable measurement system. The present invention is mainly applied to measure biological information of animal phyla, is preferably applied to vertebrates, and is more preferably applied to vertebrates living on land. The present invention is more preferably applied to quadruped animals including mammals, reptiles, and amphibians. The present invention is applicable to animals on which harnesses, belts, garments, and the like (hereinafter collectively referred to as garments) can be put.

The present inventors thought that it was important not to allow a body contact electrode to be displaced from the body owing to a movement in order to accurately measure biological information about animal phyla that move around. In view of this, the garment for measuring biological information of the present invention includes the front body and the back body having high tensile elongations EMT, and thus the body contact electrode is less likely to be displaced from the body even upon a movement of the animal. Further, the garment for measuring biological information includes the band around the torso, and thus the electrode can be prevented from being displaced from the body owing to a movement. Moreover, the connection portion between the front body and the back body of the garment for measuring biological information comprises a woven or knitted fabric that is stretchable in the direction from the head portion to the tail portion, and thus the electrode is less likely to be displaced from the body even upon a great movement. Therefore, the garment for measuring biological information of the present invention enables accurate measurement of biological information about an animal that makes a movement.

### BRIEF DESCRIPTION OF THE DRAWINGS

[Fig. 1] Fig. 1 is a development view of an inner surface (surface in contact with a body) of a garment 1 for measuring biological information.
[Fig. 2] Fig. 2 is a development view of an outer surface (surface not in contact with a body) of the garment 1 for measuring biological information.
[Fig. 3] Fig. 3 is a view of a dog wearing the garment 1 for measuring biological information.
[Fig. 4] Fig. 4 is a graph showing the time transition of the R-R interval of the electrocardiographic signal obtained when the measurement accuracy is sufficiently high. (Vertical axis: R-R interval [ms], horizontal axis: measurement time [hr])
[Fig. 5] Fig. 5 is a graph showing the time transition of the R-R interval of the electrocardiographic signal obtained when the measurement accuracy is low. (Vertical axis: R-R interval [ms], horizontal axis: measurement time [hr])

### MODE FOR CARRYING OUT THE INVENTION

Hereinafter, the present invention will be described in detail.

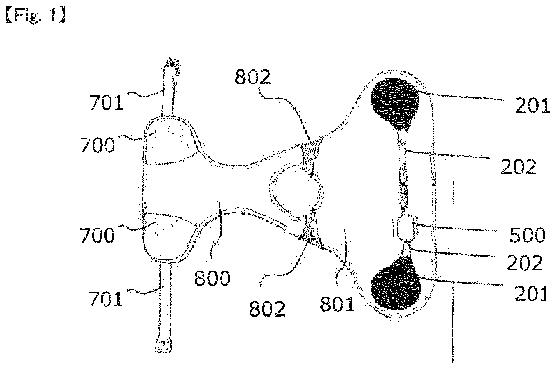

Hereinafter, an example of the configuration of the present invention will be described with reference to the drawings. FIG. 1 is a development view of a surface that is brought into contact with the body of an animal, i.e., the inner surface, of a garment for measuring biological information of the present invention. In the garment for measuring biological information, hook-and-loop fasteners 700 are formed on a front body 800. An electrode member 101 having a sheet shape and including electrode sections 201, a wiring section 202, and a snap fastener section 500 for connecting a detachable electronic unit which are integrated, is formed on a back body 801. The front body 800 and the back body 801 are connected to each other at a connection portion comprising a stretchable woven or knitted fabric 802.

In a state where an animal is dressed in the garment, the front body is a component that mainly covers the ventral side of the animal, and the back body is a component that mainly covers the dorsal side of the animal.

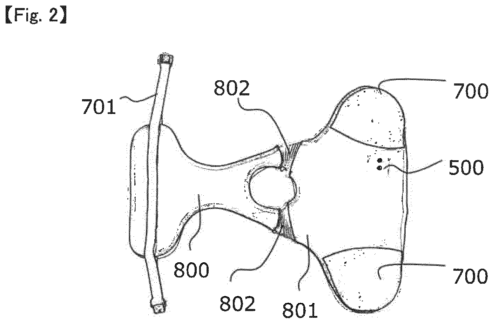

FIG. 2 is a development view of a surface that is not brought into contact with the body of an animal, i.e., the outer surface, in an example of the garment for measuring biological information of the present invention. In the garment for measuring biological information, a band 701 is attached to the front body 800. Hook-and-loop fasteners 700 and the snap fastener section 500 for connecting a detachable electronic unit are formed on the back body 801. A connection portion 802 which is the stretchable woven or knitted fabric is formed at each portion at which the front body 800 and the back body 801 are joined to each other.

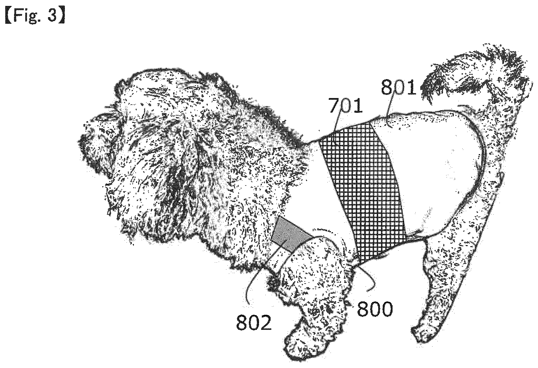

FIG. 3 illustrates a dog dressed in an example of the garment for measuring biological information of the present invention. The connection portion 802 which is the stretchable woven or knitted fabric is formed at each portion at which the front body 800 and the back body 801 are joined to each other. The band 701 is attached in a direction around the torso of the animal. FIG. 3 corresponds to a case where a thick band is attached.

Clothes used for the garment for measuring biological information of the present invention each have a longitudinal-direction tensile elongation EMT of not lower than 5% and not higher than 110% and have a lateral-direction tensile elongation EMT of not lower than 10% and not higher than 130%. It is preferable that: the longitudinal-direction tensile elongation EMT is not lower than 7% and not higher than 105%; and the lateral-direction tensile elongation EMT is not lower than 12% and not higher than 125%. It is more preferable that: the longitudinal-direction tensile elongation EMT is not lower than 10% and not higher than 100%; and the lateral-direction tensile elongation EMT is not lower than 15% and not higher than 120%. If the clothes each have a longitudinal-direction tensile elongation EMT of lower than 5% and a lateral-direction tensile elongation EMT of lower than 10%, the electrode formed on the garment for measuring biological information are displaced from the body owing to a movement of the animal, whereby biological information cannot be accurately measured. In addition, since the animal is dressed while the garment for measuring biological information is in close contact with the body such that the electrode is constantly in contact with the body, it may be difficult for the animal to make a movement, and the animal may feel uncomfortable. Meanwhile, if the clothes each have a longitudinal-direction tensile elongation EMT of higher than 110% and a lateral-direction tensile elongation EMT of higher than 130%, it is difficult to cause the electrode to be constantly in close contact with the body, whereby the electrode is displaced from the body upon a movement of the animal.

Here, the longitudinal direction refers to a direction connecting a head portion and a tail portion when an animal is dressed in the garment, and the lateral direction refers to a direction perpendicular to the longitudinal direction.

The form of a garment body of the garment for measuring biological information of the present invention is not particularly limited and is applied to the form of a garment that can be put on an animal. Although the garment for measuring biological information may be newly designed so as to be easily applied to the present invention, an existing garment for animals can also be used. An existing garment may be worn over the garment of the present invention. For each of the front body and the back body, one fabric may be used, or fabrics may be used in a superposed manner.

For each tensile elongation EMT, a sample having a length of 15 cm and a width of 15 cm was obtained by cutting, KES-FB1 manufactured by KATO TECH CO., LTD., was used to apply a one-direction stretching force at a maximum load of 50 gf/cm with a deformation speed being set to 0.2 mm/sec., and measurement was performed. From a tensile characteristics curve obtained by the measurement, a tensile elongation EMT[%] was obtained.

The garment for measuring biological information of the present invention preferably has a band around the torso, and thus the electrode can be prevented from being displaced from the body owing to a movement. The width of the band is preferably not smaller than 1 cm and not larger than 30 cm, and can be selected according to the size of the animal to be dressed in the garment. For example, in the case of a small-sized dog such as a Chihuahua, the width of the band is preferably not smaller than 1 cm and not larger than 10 cm. In the case of a large-sized dog such as a Golden Retriever, the width of the band is preferably not smaller than 2 cm and not larger than 15 cm. In the case of a large-sized animal such as a cow, the width of the band is preferably not smaller than 5 cm and not larger than 30 cm. If the width is smaller than 1 cm, the electrode cannot be prevented from being displaced from the body. Meanwhile, if the width is large, the animal may feel that it is difficult to move.

The band is preferably partially attached to the garment body by means of sewing or adhesion. The band may include a mechanism for adjusting the length thereof. Alternatively, a material having a moderate elongation percentage may be used, as necessary.

In the garment for measuring biological information of the present invention, the connection portion between the front body and the back body is preferably a woven or knitted fabric that is stretchable in the direction from the head portion to the tail portion. The present inventors found, during observation of an animal dressed in the garment, that the clothes were greatly deformed at shoulder portions, shoulder joint portions, and arm portions thereof upon a movement of the animal. Thus, in the garment for measuring biological information of the present invention, the woven or knitted fabric is more preferably formed at each shoulder portion, each shoulder joint portion, and/or each arm portion. Consequently, the electrode is less likely to be displaced from the body even upon a great movement of the animal.

The woven or knitted fabric in the present invention preferably has an elongation percentage, in the direction from the head portion to the tail portion, of not lower than 100%. If the woven or knitted fabric has an elongation percentage, in the direction from the head portion to the tail portion, of not lower than 100%, the electrode can be prevented from being displaced from the body owing to a great movement of the animal. The above-mentioned elongation percentage is more preferably not lower than 120% and further preferably not lower than 150%. The upper limit of the elongation percentage is not particularly limited, and the elongation percentage only has to be not higher than 1000% and may be not higher than 800%.

The above-mentioned elongation percentage only has to be measured as an elongation percentage defined in JIS L1096 (2010). The elongation percentage only has to be measured by a constant-speed elongation method.

The form of the above-mentioned woven or knitted fabric is not particularly limited. The form may be a woven fabric or a knitted fabric and is preferably a knitted fabric. Examples of the form include: a knitted fabric; a form in which a knitted fabric is contracted in an accordion shape; a form in which a woven or knitted fabric obtained by using synthesized fibers is pleated to have recesses and protrusions; a form obtained by knitting with use of a ribbon knitting machine; and the like. The form is more preferably a form in which a knitted fabric is contracted in an accordion shape. The form in which a knitted fabric is contracted in an accordion shape is preferably, for example, such that: a plurality of rows of regions protruding in a curved manner to a front side and a plurality of rows of regions protruding in a curved manner to a back side, are alternately continued; and a cross-sectional shape perpendicular to a longitudinal direction is a wavy shape. In the case of a knitted fabric having a form obtained by contraction in an accordion shape, when stress is applied in a body width direction, the regions protruding in a curved manner have substantially planar shapes so that the body width increases, whereas, when the stress is eliminated, the shapes of the regions having been changed to the substantially planar shapes are restored to the original curved shapes so that the body width decreases. A knitted fabric having a form obtained by contraction in an accordion shape is available from, for example, INOUE RIBBON INDUSTRY Co., Ltd., etc.

The above-mentioned woven or knitted fabric is stretchable according to a movement of the animal, but preferably does not droop at the time of dressing.

The garment for measuring biological information of the present invention is preferably such that a body contact electrode is located on the back body. The electrode sections are preferably located on the sides of the trunk and more preferably at the thorax. If the electrode is located at the thorax, the electrode is constantly in contact with the body even upon a movement of the animal, whereby biological information can be stably measured.

In the garment for measuring biological information of the present invention, a detachable electronic unit can be connected to the surface that is not brought into contact with a body, such that the electronic unit is detachable while an animal is dressed in the garment. The electronic unit may be exposed or may be equipped with a cover.

The band formed on the garment for measuring biological information of the present invention is preferably placed in the direction around the torso of the animal so as to overlap the body contact electrode and/or the electronic unit. Consequently, the electrode can be prevented from being displaced from the body owing to a movement of the animal. Further, if the band overlaps the electronic unit, the electronic unit neither falls nor is shaken owing to a movement of the animal, whereby biological information can be stably measured.

The biological information in the present invention refers to a cardiac electrical activity, a heart rate waveform, a myoelectric activity, a body temperature, the temperature in the garment, a respiratory rate, a respiratory condition, the amount of sweating, a sweating stage, a joint angle, the displacement amount of each part of the body, the acceleration rate of each part of the body, position information about each part of the body, etc. The electrode and the electronic unit are appropriately selected according to the biological information to be measured.

The present invention is preferably configured to include at least a plurality of electrodes that are brought into contact with a body or contact with a body with a conductive paste therebetween, and is preferably configured to further include an electrode capable of measuring a heart rate waveform or a cardiac electrical activity as the biological information.

In the present invention, it is preferable that a combination with a detachable electronic unit having a measurement function besides the electrode, a communication function, and the like, is made. Such a detachable electronic unit is connected to each electrode via a connector provided to the garment. Electrical wiring is provided between the connector and the electrode. In the case where the present invention is intended to mainly measure a cardiac electrical activity out of biopotentials, the present invention preferably includes: a signal processing means for converting, into an electrocardiogram signal, a body surface potential obtained from each electrode by the electronic unit; a sampling means for sampling the electrocardiogram signal obtained by the signal processing means; and a transmission means for wirelessly transmitting an electrocardiogram signal obtained by the sampling to an analyzer. The electrocardiogram signal is transmitted by the transmission means to the analyzer such as a portable terminal, a personal computer (hereinafter, referred to as a PC), or the like, and the transmitted electrocardiogram signal can be analyzed and displayed on the analyzer. For example, an analog filter or a digital filter may be used as the signal processing means, and a means such as radio wave or infrared communication may be used as the transmission means.

In general, the measurement result of cardiac electrical activity is recorded as an electrocardiogram and an electrocardiogram waveform in which the horizontal axis represents time and the vertical axis represents potential difference. The waveform that appears in the electrocardiogram and the electrocardiogram waveform for each heartbeat is mainly composed of five typical waves: P wave, Q wave, R wave, S wave, and T wave. There may be a U wave besides these waves. The wave from the start of the Q wave to the end of the S wave may be referred to as QRS wave. The electronic unit of the present invention preferably includes an electrode capable of detecting at least the R wave from among these waves. The R wave shows the activation of both the left and right ventricles and is the wave with the largest potential difference. The time from the peak of the R wave to the peak of the next R wave is generally called RR interval (RRI), and the heart rate per minute can be calculated using the equation (heart rate)=60/(RR interval (seconds)). That is, the heart rate can be found by detecting the R wave using an electrode capable of detecting the R wave. In the present invention, QRS waves are also included in R waves unless otherwise noted.

The garment for measuring biological information according to the present invention includes at least clothes, a single electrode made of a conductive material, a single-pole connector section, and a wiring that electrically connects the electrode and the connector section, the electrode and the wiring being preferably made of the same material in order to reduce a level difference and ensure connection reliability. Although not particularly limited, the garment for measuring biological information according to the present invention includes: an electrode provided on the inner surface of the garment body in contact with a living body; a connector section and an electronic unit provided on an outer surface not in contact with the living body; and a wiring that connects the electrode and the wiring. The distance between the connector and the electrode is preferably as short as possible.

In the present invention, the electrode preferably includes a conductive layer capable of detecting electrical information of a body, and further includes an insulating layer. The electrode has an area necessary for detecting electrical information of a body such as an electrocardiogram, and the area of each electrode is preferably 1 cm² or more. More preferably, it is 5 cm² or more, and still more preferably 10 cm² or more. The upper limit is not particularly limited, but is preferably 100 cm² or less. The electrode can be formed into any shape such as a rectangle, a triangle, a polygon with five or more sides, a circle, and an ellipse.

It is preferable that the electrode and/or the wiring in the present invention have stretchability so that they can follow the movement of the animal.

As the conductor layer of the electrode and/or the electrical wiring in the garment for measuring biological information according to the present invention, a metal foil, conductive fibers, conductive yarns, and a stretchable conductor layer can be used, for example. Examples of usable metal foil include copper foil, aluminum foil, stainless steel foil, and gold foil. Examples of usable conductive fibers or conductive yarns include a metal thin wire, metal-coated natural fibers or synthetic fibers, fibers made of a polymer material containing conductive filler such as metal micro particles or carbon nanofibers, and fibers coated with or impregnated with a conductive polymer. In addition, felts, fabrics, knitted fabrics, non-woven fabrics, and the like composed of these conductive fibers or conductive yarns can also be used as the conductor layer of the electrical wiring.

The stretchability can be achieved by a knit structure made of conductive fibers or conductive yarns, zig zag embroidery, or the like. A woven fabric also has stretchability in the bias direction of warp/weft. If a woven fabric, a knitted fabric, a non-woven fabric, or the like is formed of stretchable yarns, stretchability can be naturally obtained. The metal foil can be simulated by being processed into a corrugated shape.

In the present invention, stretchability can be obtained by using a sheet-shaped electrode and/or wiring formed from a stretchable conductive composition containing conductive filler and a resin having stretchability. The sheet-shaped electrode formed from the stretchable conductive composition is preferable, because components having high electrical conductivity such as metal powders can be used for the sheet-shaped electrode, by which electric resistance lower than that when a conductive polymer is used can be obtained, and thus, a weak electric signal can be detected. The electric resistance value on the electrode surface is preferably 1000Ω_{□} or less, more preferably 300Ω_{□} or less, still more preferably 100Ω_{□} or less, and most preferably 30Ω_{□} or less in terms of sheet resistance. In the sheet-shaped electrode formed from the conductive composition, the electric resistance value on the electrode surface can be suppressed to 300Ω_{□} or less.

In addition, since the electrode formed from the conductive composition has a low electric resistance value, the wiring and the electrode can be made of the same material, which is a preferable mode of the present invention. When the wiring and the electrode are made of the same material, the width of the wiring may be 1 mm or more, and more preferably 5 mm or more and 10 mm or less.

In the present invention, it is preferable to measure biological information using a garment provided with at least two or more electrodes. Although not particularly limited, the number of electrodes is preferably 2 or more and 15 or less.

The electrode used in the present invention preferably further has an insulating layer. For example, a sheet-shaped electrode including a first insulating layer and a conductor layer can be used. A sheet-shaped wiring including a first insulating layer, a conductor layer, and a second insulating layer can be used for the wiring used in the present invention.

As the mechanism for analyzing the measured information, a conventionally known analysis device (such as a heart rate meter, an electrocardiograph, or an electromyograph) according to purposes may be employed, and the mechanism includes a unit for transmitting information to an external analysis device.

The biological information measurement method and the garment for measuring biological information according to the present invention are also applicable to a technique for detecting a physiological state and a psychological state of animals on the basis of the collected biological information or in combination with a biological information measurement method and a garment for measuring biological information different from those of the present invention. Examples of such techniques include management of the psychoneurotic state of animals by measuring a degree of relaxation, and management of the health state by measuring the heart rate.

The biological information measurement method and the garment for measuring biological information according to the present invention enable measurement of biological information in such a way that the garment for measuring biological information is put on an animal, a conductive base material is applied on the electrode, and the electrode is brought into close contact with the body of the animal. Non-limiting examples of the conductive base material include a conductive gel, a conductive paste, and a conductive jelly. Examples of commercially available product of the conductive paste include singa gel (Parker Laboratories, INC) and Ten20 (Weaver and Company). The electrode in the present invention may be provided with a moisture retention layer in order to reduce contact impedance with a living body.

### EXAMPLES

Hereinafter, the present invention will be more specifically described by means of examples. However, the present invention is not limited by the following examples and can also be carried out with modifications being made within the scope of the gist described above and below, and each of these modifications are included in the technical scope of the present invention.

Tensile elongation: Test pieces each having a length of 15 cm and a width of 15 cm were obtained, and a tensile shear tester (KES-FB1-A) manufactured by KATO TECH CO., LTD., was used to obtain tensile elongations (EMT) under a condition of a tension speed of 0.2 mm/s and a maximum load of 50 gf/cm in the longitudinal direction and the lateral direction. Specifically, when the length of each test piece having received the maximum load is defined as B and the original length of the test piece is defined as A, the value of ((B-A)/A)×100 was used as a tensile elongation (%).

Elongation percentage: An elongation percentage defined in method A (constant-speed elongation method) of JIS L1096 (2010) 8.16.1 was measured.

Accuracy of heart rate measurement: A dog was dressed in a garment for measuring biological information obtained in each of the following examples and comparative examples. The dog was instructed to make movements, i.e., instructed to sit, wait, walk, and run, and an R-R interval during the movements was measured. When noise is mixed owing to displacement of the electrode upon such movements, a phenomenon in which the R-R interval is longer than 2000 ms is observed. Thus, when a time period during which the R-R interval was longer than 2000 ms was not shorter than 100 seconds per hour, the accuracy of the measurement was regarded as being insufficient and was evaluated as "×". Meanwhile, when the time period was not shorter than 20 seconds and shorter than 100 seconds per hour, the accuracy of the measurement was regarded as being low and was evaluated as "Δ". Meanwhile, when the time period was shorter than 20 seconds per hour, the accuracy of the measurement was regarded as being sufficient and was evaluated as "o".

### [Example 1]

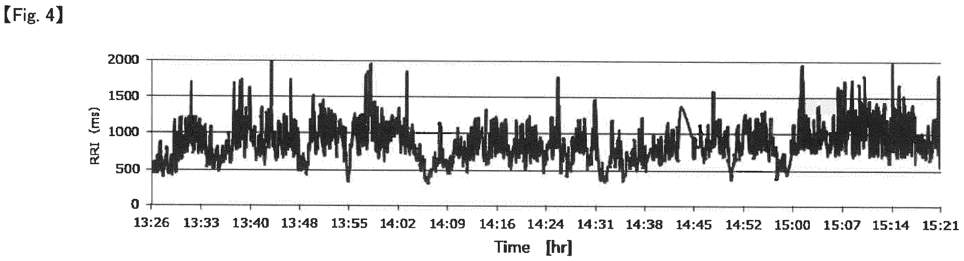

A garment for measuring biological information was manufactured by using a front body and a back body each having a longitudinal-direction tensile elongation EMT of 15% and a lateral-direction tensile elongation EMT of 26%. The form of the garment was the same as the form in each of FIG. 1 and FIG. 2, but the garment included neither a band nor a stretchable woven or knitted fabric. SIGNAGEL (Parker Laboratories, Inc.) was applied on the electrode, a Chihuahua was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was stably measured even upon great movements. FIG. 4 shows the measurement result of the R-R interval.

### [Example 2]

A garment for measuring biological information was manufactured by using a front body and a back body each having a longitudinal-direction tensile elongation EMT of 36% and a lateral-direction tensile elongation EMT of 46%. The form of the garment was the same as the form in each of FIG. 1 and FIG. 2. The garment included the front body having a band with a width of 5 cm, but the garment did not include a stretchable woven or knitted fabric. SIGNAGEL (Parker Laboratories, Inc.) was applied on the electrode, a Maltese was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was stably measured even upon great movements.

### [Example 3]

A garment for measuring biological information was manufactured by using a front body and a back body each having a longitudinal-direction tensile elongation EMT of 73% and a lateral-direction tensile elongation EMT of 94%. The form of the garment was the same as the form in each of FIG. 1 and FIG. 2. The garment included a stretchable woven or knitted fabric as a connection portion between the front body and the back body, but the garment did not include a band. SIGNAGEL (Parker Laboratories, Inc.) was applied on the electrode, a Maltese was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was stably measured even upon great movements.

### [Example 4]

A garment for measuring biological information was manufactured by using a front body and a back body each having a longitudinal-direction tensile elongation EMT of 15% and a lateral-direction tensile elongation EMT of 26%. The form of the garment was the same as the form in each of FIG. 1 and FIG. 2. The garment included the front body having a band with a width of 8 cm, and the garment include a stretchable woven or knitted fabric as a connection portion between the front body and the back body. SIGNAGEL (Parker Laboratories, Inc.) was applied on the electrode, a Shiba inu was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was stably measured even upon great movements.

### [Example 5]

A garment for measuring biological information was manufactured by using a front body and a back body each having a longitudinal-direction tensile elongation EMT of 27% and a lateral-direction tensile elongation EMT of 34%. The form of the garment was the same as the form in each of FIG. 1 and FIG. 2. The garment included the front body having a band with a width of 10 cm, and the garment include a stretchable woven or knitted fabric as a connection portion between the front body and the back body. SIGNAGEL (Parker Laboratories, Inc.) was applied on the electrode, a Golden retriever was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was stably measured even upon great movements.

### [Example 6]

A garment for measuring biological information was manufactured by using a front body and a back body each having a longitudinal-direction tensile elongation EMT of 86% and a lateral-direction tensile elongation EMT of 105%. The form of the garment was the same as the form in each of FIG. 1 and FIG. 2. The garment included the front body having a band with a width of 10 cm, and the garment include a stretchable woven or knitted fabric as a connection portion between the front body and the back body. SIGNAGEL (Parker Laboratories, Inc.) was applied on the electrode, a Golden retriever was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was stably measured even upon great movements.

### [Example 7]

A garment for measuring biological information was manufactured by using a front body having a longitudinal-direction tensile elongation EMT of 36% and a lateral-direction tensile elongation EMT of 46%, and a back body having a longitudinal-direction tensile elongation EMT of 15% and a lateral-direction tensile elongation EMT of 26%. The form of the garment was the same as the form in each of FIG. 1 and FIG. 2. The garment included the front body having a band with a width of 10 cm, and the garment include a stretchable woven or knitted fabric as a connection portion between the front body and the back body. SIGNAGEL (Parker Laboratories, Inc.) was applied on the electrode, a Golden retriever was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was stably measured even upon great movements.

### [Comparative example 1]

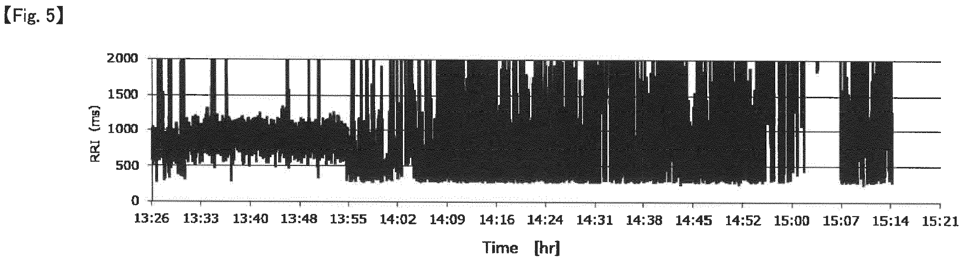

A garment for measuring biological information was manufactured by using a front body and a back body each having a longitudinal-direction tensile elongation EMT of 2% and a lateral-direction tensile elongation EMT of 1%. The form of the garment was the same as the form in each of FIG. 1 and FIG. 2. The garment included the front body having a band with a width of 5 cm, and the garment include a stretchable woven or knitted fabric as a connection portion between the front body and the back body. SIGNAGEL (Parker Laboratories, Inc.) was applied on the electrode, a Maltese was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was not stably measured becouese a lot of noise enters upon great movements. FIG. 5 shows the measurement result of the R-R interval.

### [Comparative example 2]

A garment for measuring biological information was manufactured by using a front body and a back body each having a longitudinal-direction tensile elongation EMT of 2% and a lateral-direction tensile elongation EMT of 1%. The form of the garment was the same as the form in each of FIG. 1 and FIG. 2. The garment included the front body having a band with a width of 10 cm, but the garment did not include a stretchable woven or knitted fabric. SIGNAGEL (Parker Laboratories, Inc.) was applied on the electrode, a Golden retriever was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was not stably measured becouese a lot of noise enters upon great movements.

### [Comparative example 3]

A garment for measuring biological information was manufactured by using a front body and a back body each having a longitudinal-direction tensile elongation EMT of 120% and a lateral-direction tensile elongation EMT of 135%. The form of the garment was the same as the form in each of FIG. 1 and FIG. 2. The garment included the front body having a band with a width of 10 cm, and the garment include a stretchable woven or knitted fabric as a connection portion between the front body and the back body. SIGNAGEL (Parker Laboratories, Inc.) was applied on the electrode, a Maltese was dressed in the garment as shown in FIG. 3, and the R-R interval in daily life was measured by using WHS-1 (manufactured by UNION TOOL Co.) as an electronic unit. Table 1 indicates longitudinal-direction tensile elongations EMT and lateral-direction tensile elongations EMT of the garment for measuring biological information, the width of the band used, presence/absence of a stretchable woven or knitted fabric, and the accuracy of the heart rate measurement. The R-R interval was not stably measured becouese a lot of noise enters upon great movements.

**[Table 1]**

| | Front body | | Back body | | Band (cm) | Stretchable woven or knitted fabric | Accuracy of measurement |
|---|---|---|---|---|---|---|---|
| | Tensile elongation EMT (%) | | Tensile elongation EMT (%) | | | | |
| | longitudinal-direction | lateral-direction | long itud inal-d irection | lateral-direction | | | |
| Example 1 | 15 | 26 | 15 | 26 | - | - | Δ |
| Example 2 | 36 | 46 | 36 | 46 | 5 | - | Δ |
| Example 3 | 73 | 94 | 73 | 94 | - | Included | Δ |
| Example 4 | 15 | 26 | 15 | 26 | 8 | Included | ○ |
| Example 5 | 27 | 34 | 27 | 34 | 10 | Included | ○ |
| Example 6 | 86 | 105 | 86 | 105 | 10 | Included | ○ |
| Example 7 | 36 | 46 | 15 | 26 | 10 | Included | ○ |
| Comparative example 1 | 2 | 1 | 2 | 1 | 5 | Included | × |
| Comparative example 2 | 2 | 1 | 2 | 1 | 10 | - | × |
| Comparative example 3 | 120 | 135 | 120 | 135 | 10 | Included | × |

### INDUSTRIAL APPLICABILITY

The present invention makes it possible to provide: a garment for measuring biological information, enabling an R-R interval to be stably measured even upon a great movement of an animal; and a biological information measurement method. A sympathetic nervous system activity serving as an index of stress and a parasympathetic nervous system activity serving as an index of relaxation can be analyzed from the R-R interval of the animal. Thus, mental inference for the animal can be performed. Therefore, it becomes easy to develop: clothes, laying materials, gauzes, and the like that enable an animal to be relaxed; and feed, snacks, and the like that an animal prefers. In addition, although quarantine detector dogs, police dogs, and the like communicate abnormalities only by means of actions and barking, it becomes possible to detect abnormalities from a change in the R-R interval. Thus, the present invention is expected to make great industrial contributions.

### DESCRIPTION OF REFERENCE SIGNS

- 101:: electrode member having a sheet shape
- 201:: electrode sections
- 202:: wiring section
- 500:: snap fastener section
- 700:: hook-and-loop fasteners
- 701:: band
- 800:: front body
- 801:: back body
- 802:: stretchable woven or knitted fabric

## Claims

1. A garment for measuring biological information for animals, the garment comprising
a body contact electrode,
a front body of the garment for measuring biological information, and
a back body of the garment for measuring biological information, wherein
the front body and/or the back body has a longitudinal-direction tensile elongation EMT of not lower than 5% and not higher than 110% and has a lateral-direction tensile elongation EMT of not lower than 10% and not higher than 130%.

2. The garment for measuring biological information according to claim 1, wherein
the front body and the back body of the garment for measuring biological information are connected to each other at a connection portion comprising a stretchable woven or knitted fabric.

3. The garment for measuring biological information according to claim 2, wherein
the woven or knitted fabric has an elongation percentage in a direction from a head portion to a tail portion, the elongation percentage being not lower than 100%.

4. The garment for measuring biological information according to any one of claims 1 to 3, wherein
the woven or knitted fabric is disposed at at least one portion of the garment for measuring biological information, the one portion being selected from among a shoulder portion, a shoulder joint portion, and an arm portion.

5. The garment for measuring biological information according to any one of claims 1 to 4, wherein
the body contact electrode is located on a side of the back body of the garment for measuring biological information, and the side is brought into contact with a body.

6. The garment for measuring biological information according to any one of claims 1 to 5, the garment further comprising
a band, wherein
the band has a width of not smaller than 1 cm and not larger than 30 cm, and the band is attached in a direction around a torso of an animal.

7. The garment for measuring biological information according to claim 6, wherein
the band is disposed on an outer side of the garment at a location of the body contact electrode.

8. The garment for measuring biological information according to any one of claims 1 to 7, wherein
the body contact electrode is located at a thorax in the garment for measuring biological information.

9. The garment for measuring biological information according to any one of claims 1 to 8, wherein
biological information to be measured is an R-R interval which is an interval between an R wave and an R wave in an electrocardiogram.

10. A biological information measurement method comprising dressing an animal in the garment for measuring biological information according to any one of claims 1 to 9, to measure biological information.

11. The biological information measurement method according to claim 10, further comprising applying a conductive base to the body contact electrode.
